# EUROPEAN PATENT SPECIFICATION

(11) **EP 3 829 498 B1**
(45) Date of publication and mention of the grant of the patent: **23.07.2025**
(21) Application number: 18749777.1
(22) Date of filing: 01.08.2018
(51) Int. Cl.: A61F 7/02

(54) **A HEALTH PROMOTING DEVICE**
GESUNDHEITSFÖRDERNDE VORRICHTUNG
DISPOSITIF FAVORISANT LA SANTÉ

(43) Date of publication of application: 09.06.2021
(73) Proprietor: Ergostone Ab, 837 97 Åre (SE)
(72) Inventor: STRÖM, Viktor, 837 97 Åre (SE)
(74) Representative: AWA Sweden AB
(86) International application number: PCT/EP2018/070820
(87) International publication number: WO 2020/025123

(56) References cited:
- EP-A1- 1 925 273
- EP-A1- 2 145 612
- CA-A- 1 200 731
- CN-U- 202 875 576
- DE-A1- 19 808 316
- GB-A- 2 373 322
- JP-B2- 5 210 747
- US-A- 2 595 328
- US-A- 5 897 580
- US-A- 640 534
- US-A1- 2012 316 626
- US-A1- 2014 231 280

## Description

### FIELD OF THE INVENTION

The present invention relates generally to a health promoting device and more specifically to a health promoting device that comprises a bag filled with compressible material.

### BACKGROUND OF THE INVENTION

The urge for humans to promote their health by working out, curing both physical and psychological symptoms, having fun and so on is a fundamental drive. This in turn makes the health promoting industry flourish. The market is flooding with more or less expensive health promoting products, each making a different promise to the user, but which products many times end up forgotten in the wardrobe. There is a need for a long life time, high quality health promoting device that can be used for many different health promoting applications, thereby decreasing the need for the user to purchase a plurality of different products. US640534, US2595328, EP1925273, EP2145612, CA1200731 and US2014/231280 are considered relevant prior art for the present application.

### SUMMARY OF THE INVENTION

It is an object of the present invention to at least provide an improved health promoting device, which can be used for various health promoting applications and activities. This object is achieved by a device of the present invention as defined in the appended independent claim. Preferred embodiments are set forth in the dependent claims and in the following description and drawings.

Thus, in accordance with the present inventive concept, there is provided a health promoting device comprising a bag having a sealed cavity which is at least partly filled with soapstone powder. Filling the bag with soapstone powder provides a number of advantages of the device. The device has a high heat retention. It can be used in room temperature or in a heated up or cooled down state. The device according to the present inventive concept may thus be used as a health-, wellness-, recreation- or training product which can advantageously be combined with a heating or cooling effect when desired since the soapstone powder has a thermal conductivity and specific heat capacity due to its mineral composition which facilitates for its use as a warming/cooling tool for human use as a health promoting device. Soapstone powder has a relatively high bulk density, which makes the device heavy with respect to its volume, which is useful for providing weight or pressure on a body part to e.g. exercise a muscle group or to provide a comforting feeling which suppresses anxiety. Further, since soapstone has an excellent chemical durability and purity it is suitable for human use. Due to its powder structure, which provides a flowabilty and compressibility to the soapstone material, the device can be formed into various shapes. The device can be used to support the treatment of e.g. anxiety, tension, back pain, Ischia's, and improve sleeping and blood circulation among other things. For athletes or health care patients the device can be used as cold therapy of acute cooling of injuries, also out on the field, or for heat therapy prior to an activity to loosen up muscles and cold therapy afterwards. This is a non-exhaustive list of applications that are suitable for the present inventive concept. Many other applications are conceivable.

According to the invention, the bag is made of elastomer, wherein said elastomer is silicone. By forming the bag from a silicone rubber the wall of the bag is effectively sealed such that no soapstone powder can escape from the device. Further, the elastomer bag is elastic such that the device may be bent around e.g. body parts, be squeezed by the hand, or compressed to provide support to e.g. the neck. By selecting an elastomer with a high deformability the device can be formed to a desired shape applicable to a desired use. Further advantages with an elastomer bag is that the bag can be manufactured into a desired shape, e.g. by molding the elastomer material into the desired shape. Elastomer silicone is further useful as it has a long lifetime and can withstand wide range of temperatures and keep its physical characteristics over a large temperature range (covering using the device after being cooled down in a freezer, room temperature, and after it has been heated up in e.g. a micro wave oven).

According to an embodiment of the device, the bag is water tight when sealed, which is advantageous to protect the soapstone powder from moist, thereby extending its use to moist environments.

According to an embodiment of the device, the bag has no seams, which is advantageous for a pleasant experience for the user.

According to an embodiment of the device, the device has one of a substantially rectangular-, circular-, and oval cross-section.

According to an embodiment of the device, the device is elongated, which is advantageous. The elongated shape may be a straight or a bent shape. Other shapes of the device, like e.g. rectangular-, disc-, spherical shape or the like, are applicable within the inventive concept and is preferably selected based on the specific intended application of the device.

According to an embodiment of the device, the bag is sealed by means of one of a knot, glue, and (chemical) welding.

According to an embodiment of the device, it further comprises a sealing mechanism for sealing the bag, thereby containing the soapstone powder in the bag. The sealing mechanism may be arranged at an end portion of the bag which is advantageous when filling the bag with soapstone powder.

According to an embodiment of the device, the sealing mechanism and the bag are molded together in a single material, i.e. the sealing mechanism may be an integrated seal, which is advantageous. The bag and/or sealing mechanism may be molded as one piece in a (compression) mold, which advantageously provides a single manufacturing step. Molding of the bag is advantageous because creating different shapes of the bag is facilitated.

According to an embodiment of the device, the sealing mechanism comprises protrusions and corresponding receiving apertures, which when pressed together form a seal to close/seal the bag.

According to an embodiment of the device, the sealing mechanism is a clamping device.

According to an embodiment of the device, a low air pressure is applied inside said filled cavity.

According to an embodiment of the device, it further comprises an outer shell, which can advantageously be adapted to different applications of the device. The outer shell may be removably arranged, be made of any material which is adaptable to the intended use of the device, such as fabric, fur fabric, fur, plastic, etc. Further, it is conceivable that the outer shell is provided with decorations and so on.

According to an embodiment of the device, it further comprises at least one handle either arranged directly on the bag or as part of the outer shell, which is advantageous because the device can be adapted to fit a certain application e.g. to act as a (kettlebell) weight.

These and other aspects, features, and advantages of the invention will be apparent from and elucidated with reference to the embodiments described hereinafter.

### BRIEF DESCRIPTION OF THE DRAWINGS

The invention will now be described in more detail and with reference to the appended drawings in which:
Figs. 1a - 1f illustrate an exemplifying embodiment of a health promoting device according to the present inventive concept in which
   Fig. 1a is a schematic perspective upper side view,
   Fig. 1b is a schematic side view,
   Fig. 1c is a schematic end view,
   Fig. 1d is a schematic side view marking out two cross sections in the embodiment,
   Fig. 1e is a schematic first cross sectional view along a longitudinal direction of the exemplifying embodiment, and
   Fig. 1f is a schematic second cross sectional view in the cross direction of the exemplifying embodiment;
Figs. 2a to 2c are schematic cross sectional views in the cross direction of a respective embodiment of a health promoting device according to the present inventive concept;
Fig. 3 is a flow chart of a process for manufacturing a health promoting device according to the present inventive concept; and
Fig. 4 and Fig. 5 are schematic illustrations of embodiments of the inventive concept.

### DESCRIPTION OF PREFERRED EMBODIMENTS

The present invention will now be described more fully hereinafter with reference to the accompanying drawings. The below embodiments are provided by way of example so that this disclosure will be thorough and complete, and will fully convey the scope of the invention to those skilled in the art. Like numbers refer to like elements throughout.

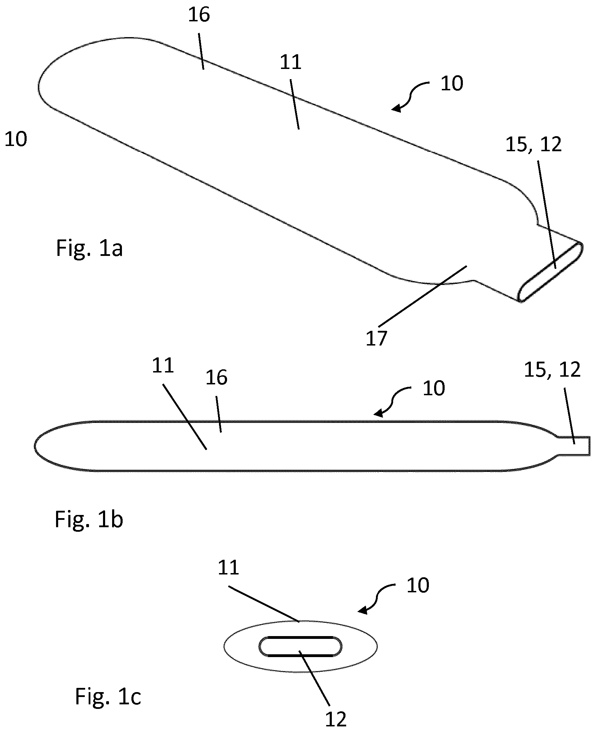

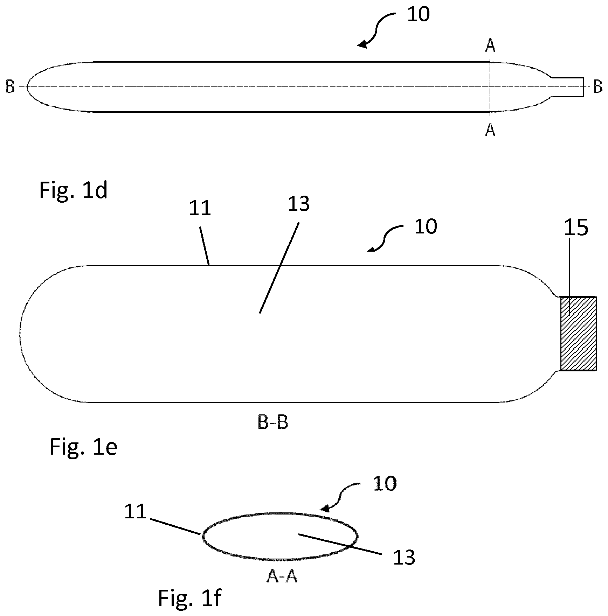

Fig. 1 illustrates an exemplifying embodiment of a health promoting device according to the present inventive concept. With reference now to Figs. 1a and 1b a health promoting device 10, which for simplicity is herein after referred to as the device 10, comprises an elongated body 16 which is substantially rectangular with an oval cross-section, see Fig. 1c, which is constituted by a bag 11 which is filled with a filling material in powder form, and more specifically with soapstone powder. Soapstone is known under many names such as e.g. soapstone, massive talc, French chalk, soapstone silicate, steatite, soapstone talc, natural steatite talc, CAS# 14807-96-6. Soapstone generally refers to all massive gray to bluish or greenish talcose rocks which have a slippery feeling, is very soft and can be carved by hand. Soapstone powder is obtained by grinding soapstone. The bag 11 is formed from an elastomer material like silicon. An opening 15 for receiving the filling material when manufacturing the device is here arranged at a first end portion 17 of the bag 11. Other positions of the opening of the bag are feasible, the positions depending on e.g. shape of the bag, manufacturing process etc.

According to an embodiment of the device, the bag 11 is waterproof.

To continue, in this embodiment, a sealing mechanism 15 is arranged at the opening 12 to seal the device 10 after filling it with the soapstone stone powder. The sealing mechanism illustrated schematically in Fig. 1a and Fig. 1e, the latter which shows a cross-sectional view along the longitudinal axis of the device 10, here refers to a stopper 15. However, a sealing of the bag is according to an embodiment provided as an integrated part of the bag, e.g. by molding or 3D-printing it as part of the bag, and preferably in a single material, e.g. silicone. The sealing mechanism may further be embodied by a separate or integrated clamping device, which may be made of e.g. polypropylene, polyethylene, polyvinylchloride or some other applicable material.

According to an embodiment of the device, the sealing mechanism is a clamp provided with protrusions and corresponding receiving apertures facing each other e.g. in a clamp, which when pressed together form the seal to close the bag.

According to an embodiment of the device, forming of the sealing mechanism/sealing the bag is an integrated step of the manufacturing of the bag, such that when the bag has been filled with soapstone powder, the bag is sealed in such a manner that a bag has no seams.

According to another embodiment, the bag is sealed by means of a knot, glue, or chemical welding.

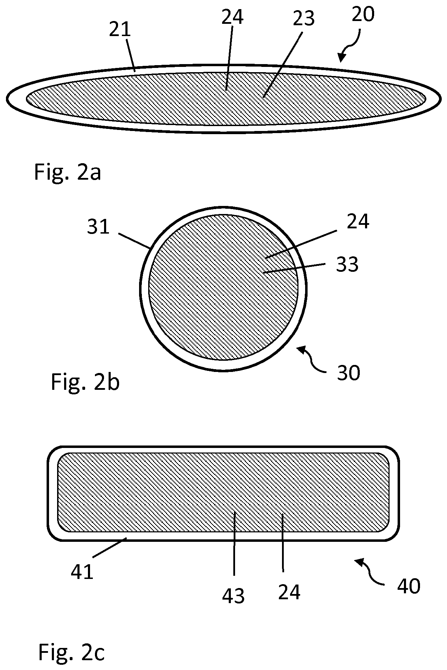

When filled with soapstone, a low air pressure is optionally applied inside said filled cavity before sealing the bag. The device 10 described with reference to Fig. 1 is substantially elongated, rectangular straight shape, and has an oval cross section. It should be noted that according to different embodiments, the device has other shapes: e.g. elongated bent shape, a disc shape, square shape, spherical shape or other applicable shape. The different shapes may further have substantially rectangular-, circular-, or oval cross-sections, see Figs. 2a-2c which illustrate cross-sections of three embodiments of the device.

Referring now to Fig. 2a, a device 20 comprises a bag 21, preferably made of silicon rubber, having a cavity 23 which is filled with soapstone powder 24, where the cross-section of the bag 21 is oval. The device 20 may be embodied by e.g. a straight or bent elongated substantially rectangular- or oval shape, or a disc shape.

In Fig. 2b, a device 30 comprises a bag 31, preferably made of silicon rubber, having a cavity 33 which is filled with soapstone powder 24, where the cross-section of the bag 31 is circular. The device 30 may be embodied by e.g. a straight or bent elongated substantially tubular- or spherical shape.

In Fig. 2c, a device 40 comprises a bag 41, preferably made of silicon rubber, having a cavity 43 which is filled with soapstone powder 24, where the cross-section of the bag 41 is substantially rectangular. The device 40 may be embodied by e.g. a straight or bent elongated substantially rectangular- or square shape. Other shapes are applicable within the present inventive concept.

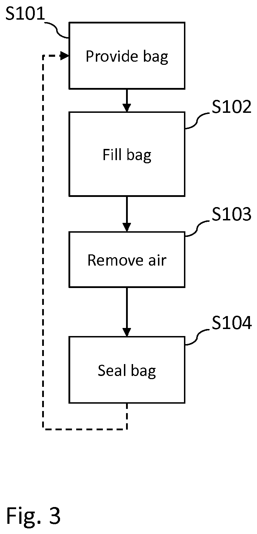

With reference now to Fig. 3, a device according to the present inventive concept is generally manufactured by performing the steps of: manufacturing or providing the bag, S101, e.g. by molding the bag in an elastomeric material like silicone. The bag is then filled with soapstone powder, S102. Optionally, a step for removing air from the bag, S103, is performed to keep the amount of air remaining inside the bag after the bag has been filled with the soapstone powder to a minimum. In a next step, S104, the bag is sealed, e.g. with a sealing mechanisms as described above, or alternatively by means of glue, chemical welding etc.

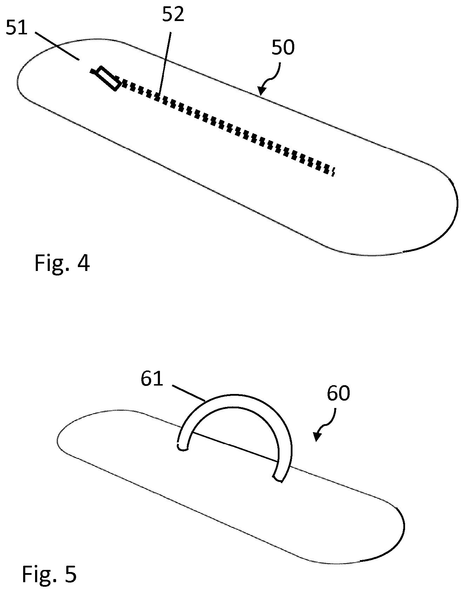

According to an embodiment of the device as schematically illustrated in Fig. 4, the device 50 further comprises an outer shell 51, which may be adapted to protect the device, provide a desired appearance or the like. The outer shell may be made in any desired material like a fabric or silicone. In the exemplifying embodiment in Fig. 4, the outer shell 51 has means for opening and reclosing the outer shell, here a zipper 52, such that the outer shell can be taken off to be cleaned or exchanged for another outer shell. Other means for opening and reclosing the outer shell are conceivable, e.g. hook- and loop fasteners etc. As schematically illustrated in Fig. 5, according to an embodiment of the device, the device 60 may further comprise one or more handles 61, arranged either directly on the bag or as part of the outer shell.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measured cannot be used to advantage. Any reference signs in the claims should not be construed as limiting the scope.

## Claims

1. A health promoting device (10) comprising a bag (11) enclosing a single sealed cavity (13) which is filled with soapstone powder and sealed with a minimum amount of air in the bag wherein said bag is made of elastomer, and wherein said elastomer is a silicone material.

2. A device according to any preceding claim, wherein said bag is water tight.

3. A device according to any preceding claim, wherein said bag has no seams.

4. A device according to any preceding claim, wherein said device has one of a substantially rectangular-, circular-, and oval cross-section.

5. A device according to any preceding claim, wherein said device has one of an elongated straight shape, an elongated bent shape, a rectangular shape, a disc shape and a spherical shape.

6. A device according to any preceding claim, wherein said bag is sealed by means of one of a knot, glue, and chemical welding.

7. A device according to any preceding claim, further comprising a sealing mechanism for sealing said soapstone powder in said bag.

8. A device according to claim 8, wherein said sealing mechanism and said bag are molded together in a single material.

9. A device according to any of claims 8 or 9, wherein said sealing mechanism comprises protrusions and corresponding receiving apertures, which when pressed together form a seal to close said bag.

10. A device according to any of claims 8 to 10, wherein said sealing mechanism is a clamping device.

11. A device according to any preceding claim, further comprising an outer shell.

12. A device according to any preceding claim, further comprising at least one handle.

13. A method of manufacturing a device according to any preceding claim comprising:
providing the bag;
filling the single cavity with soapstone powder;
removing air from the single cavity to minimize the amount of air in the bag and sealing the bag.

14. A method according to claim 13, wherein the step of providing the bag is performed by means of molding the bag or 3D printing the bag.

15. A method according to claim 13 or 14, wherein the step of sealing the bag is performed by means of chemical welding, gluing, applying a knot, or applying a clamping.

## Patentansprüche

1. Gesundheitsfördernde Vorrichtung (10), umfassend einen Beutel (11), der einen einzigen versiegelten Hohlraum (13) umschließt, der mit Specksteinpulver gefüllt und mit einer minimalen Luftmenge in dem Beutel versiegelt ist,
wobei der Beutel aus Elastomer hergestellt ist und
wobei das Elastomer ein Silikonmaterial ist.

2. Vorrichtung nach einem der vorhergehenden Ansprüche, wobei der Beutel wasserdicht ist.

3. Vorrichtung nach einem der vorhergehenden Ansprüche, wobei der Beutel keine Nähte aufweist.

4. Vorrichtung nach einem der vorhergehenden Ansprüche, wobei die Vorrichtung einen im Wesentlichen rechteckigen, kreisförmigen oder ovalen Querschnitt aufweist.

5. Vorrichtung nach einem der vorhergehenden Ansprüche, wobei die Vorrichtung eine längliche gerade Form, eine längliche gebogene Form, eine rechteckige Form, eine Scheibenform oder eine kugelförmige Form aufweist.

6. Vorrichtung nach einem der vorhergehenden Ansprüche, wobei der Beutel durch einen Knoten, Klebstoff oder chemisches Schweißen versiegelt ist.

7. Vorrichtung nach einem der vorhergehenden Ansprüche, ferner umfassend einen Versiegelungsmechanismus zum Versiegeln des Specksteinpulvers in dem Beutel.

8. Vorrichtung nach Anspruch 8, wobei der Versiegelungsmechanismus und der Beutel zusammen in einem einzigen Material geformt sind.

9. Vorrichtung nach einem der Ansprüche 8 oder 9, wobei der Versiegelungsmechanismus Vorsprünge und entsprechende Aufnahmeöffnungen umfasst, die beim Zusammendrücken eine Versiegelung zum Verschließen des Beutels bilden.

10. Vorrichtung nach einem der Ansprüche 8 bis 10, wobei der Versiegelungsmechanismus eine Klemmvorrichtung ist.

11. Vorrichtung nach einem der vorhergehenden Ansprüche, ferner umfassend eine Außenhülle.

12. Vorrichtung nach einem der vorhergehenden Ansprüche, ferner umfassend mindestens einen Griff.

13. Verfahren zum Herstellen einer Vorrichtung nach einem der vorhergehenden Ansprüche, umfassend:
Bereitstellen des Beutels,
Füllen des einzigen Hohlraums mit Specksteinpulver,
Entfernen von Luft aus dem einzigen Hohlraum, um die Luftmenge in dem Beutel zu minimieren, und Versiegeln des Beutels.

14. Verfahren nach Anspruch 13, wobei der Schritt des Bereitstellens des Beutels durch Formen des Beutels oder 3D-Drucken des Beutels durchgeführt wird.

15. Verfahren nach Anspruch 13 oder 14, wobei der Schritt des Versiegelns des Beutels durch chemisches Schweißen, Kleben, Anbringen eines Knotens oder Aufbringen einer Klemmung durchgeführt wird.

## Revendications

1. Dispositif de promotion de la santé (10) comprenant un sac (11) renfermant une seule cavité scellée (13) qui est remplie de poudre de stéatite et scellée avec une quantité minimale d'air dans le sac
ledit sac étant en élastomère, et
ledit élastomère étant un matériau de silicone.

2. Dispositif selon n'importe quelle revendication précédente, ledit sac étant étanche.

3. Dispositif selon n'importe quelle revendication précédente, ledit sac n'ayant pas de coutures.

4. Dispositif selon n'importe quelle revendication précédente, ledit dispositif ayant l'une d'une section transversale sensiblement rectangulaire, circulaire ou ovale.

5. Dispositif selon n'importe quelle revendication précédente, ledit dispositif ayant l'une des formes suivantes : forme droite allongée, forme coudée allongée, forme rectangulaire, forme de disque et forme sphérique.

6. Dispositif selon n'importe quelle revendication précédente, ledit sac étant scellé au moyen de l'un d'un nœud, de colle et d'une soudure chimique.

7. Dispositif selon n'importe quelle revendication précédente, comprenant en outre un mécanisme de scellement pour sceller ladite poudre de stéatite dans ledit sac.

8. Dispositif selon la revendication 8, ledit mécanisme de scellement et ledit sac étant moulés ensemble dans un seul matériau.

9. Dispositif selon l'une quelconque des revendications 8 ou 9, ledit mécanisme de scellement comprenant des protubérances et des ouvertures de réception correspondantes, qui, lorsqu'elles sont pressées ensemble, forment un joint pour fermer ledit sac.

10. Dispositif selon l'une quelconque des revendications 8 à 10, ledit mécanisme de scellement étant un dispositif de serrage.

11. Dispositif selon l'une quelconque des revendications précédentes, comprenant en outre une enveloppe extérieure.

12. Dispositif selon n'importe quelle revendication précédente, comprenant en outre au moins une poignée.

13. Procédé de fabrication d'un dispositif selon n'importe quelle revendication précédente, comprenant :
la fourniture du sac ;
le remplissage de la cavité unique avec de la poudre de stéatite ;
le retrait de l'air de la cavité unique afin de minimiser la quantité d'air dans le sac et le scellement du sac.

14. Procédé selon la revendication 13, l'étape de fourniture du sac étant réalisée au moyen d'un moulage du sac ou d'une impression 3D du sac.

15. Procédé selon la revendication 13 ou 14, l'étape de scellement du sac étant réalisée au moyen d'une soudure chimique, d'un collage, de l'application d'un nœud, ou de l'application d'un serrage.
